(19) Europäisches Patentamt
European Patent Office
Office européen des brevets

(11) Publication number: **0 048 626**
**B1**

(12) # EUROPEAN PATENT SPECIFICATION

(45) Date of publication of the patent specification: **18.04.84**

(51) Int. Cl.[3]: **A 23 K 1/16**

(21) Application number: **81304365.0**

(22) Date of filing: **22.09.81**

(54) **Livestock feed.**

(30) Priority: **22.09.80 US 189540**

(43) Date of publication of application: **31.03.82 Bulletin 82/13**

(45) Publication of the grant of the patent: **18.04.84 Bulletin 84/16**

(84) Designated Contracting States: **AT BE CH DE FR GB IT LI LU NL SE**

(56) References cited: **US - A - 2 438 353**

(73) Proprietor: **SMITHKLINE BECKMAN CORPORATION, 1 Franklin Plaza, Philadelphia Pennsylvania 19101 (US)**

(72) Inventor: **Hedde, Richard Duane, 1548 Washington Lane, West Chester Pennsylvania 19380 (US)**
Inventor: **Parish, Roger Cook, 184 Redwood Road, King of Prussia Pennsylvania 19406 (US)**

(74) Representative: **Johnston, Walter Paul et al, P.O. Box 39, Welwyn Garden City Hertfordshire AL7 1EY (GB)**

**EP 0 048 626 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

ACTORUM AG

# Livestock feed

This invention comprises novel animal feed compositions as well as premixes for preparing said animal feed compositions useful for improving the food utilization and growth rate of meat producing animals which use as an active ingredient one or more chemical compounds having histamine $H_2$ receptor antagonist activity.

Compounds having histamine $H_2$ antagonist activity are a relatively new group of compounds which are useful in treating gastric and peptic ulcers in humans. Their activity is primarily due to an antagonistic effect at the histamine $H_2$ receptor centers which control acid secretion in the gastrointestinal tract. Usually, but not always, these compounds do not possess antagonistic activity against histamine $H_2$ receptors the end result of which is traditional antihistamine activity such as anti-allergic activity.

In the last decade since these compounds with their new pharmacodynamic use were found, many patents and scientific publications have become available to the public describing various chemical classes of compounds having $H_2$ antagonist activity. To date only one compound, cimetidine, U.S. Patent No. 3 950 333, has been marketed to treat ulcers in humans due to its histamine $H_2$ antagonist activity. Earlier metiamide, U.S. Patent No. 3 950 353, was studied in humans as an anti-ulcer agent for oral administration. Even earlier burimamide, U.S. Patent No. 3 808 336 was described to be active primarily after parenteral injection.

Although U.S. Patent No. 2 438 353 discloses a method of controlling the fattening rate of livestock and poultry by administering substances of low toxicity containing the thiourelyene radical –NH.CS.NH–, no compound having histamine $H_2$ antagonist activity has been described to be useful to enhance the food utilization of meat producing animals.

This invention therefore comprises new feedstuffs for meat producing animals which have dispersed uniformly therein a quantity of one or more chemical compounds which have histamine $H_2$ antagonist activity said quantity being effective for increasing food utilization but not overtly toxic to the animals. It also comprises a method for improving the efficiency of food utilization and the growth rate of livestock per unit of feed. Such feedstuffs may comprise supplemented complete or basal animal feeds or, alternatively, premixes for preparing such feeds.

The carrier or basal feed is any used in the livestock industry usually hay or corn derived but may include dried fermentation residue, alfalfa, cottonseed, barley meal, soybean meal, corn meal, rice hulls, molasses, mineral salts, vitamins, silages, beet pulp, citrus pulp, fish meal, oats, rice bran, milo, sesame meal, milk or other standard animal feed ingredients. In general therefore the feedstuff carrier may be a partial or complete feed as well as a roughage composition.

Also included for commercial purposes are premix compositions in which the active ingredient is mixed in high concentration with a carrier ingredient which is usually desirable in the complete feed such as soybean meal, corn oil, ground corn, barley, mineral mixtures such as vermiculite or diatomaceous earth, corn gluten meal, corn distillers solubles or soyflour. Such premixes may often be marketed in 10–50 lb (4.5–22.7 kg) bags and have in the present invention, a preferred concentration of about 0.5–3% by weight of active intredient base. Preferably the premix carrier is grain or grain derived. The supplemented premix is mixed with a complete feed, spread over animal feed or dissolved in drinking water.

The active chemical ingredient of the animal feed composition described above may be any compound which has demonstrable histamine $H_2$ antagonist activity which results in improved food utilization at a feed level which is nontoxic or detrimental to the animal. Examples of the chemical compounds useful in this way are:

meti amide

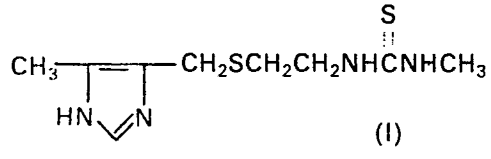

U.S. 3,950,353

(I)

cimetidine

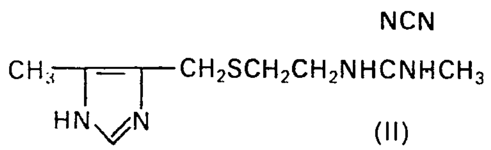

U.S. 3,950,333

(II)

ranitidine

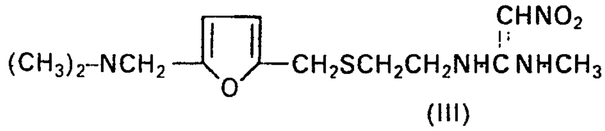

U.S. 4,128,658

(III)

N-cyano-N'-methyl-N"-[2-(5-dimethylamino-methyl-2-furanylmethylthio)ethyl]guanidine

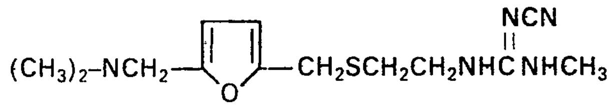

U.S. 4,128,658

(IV)

tiotidine

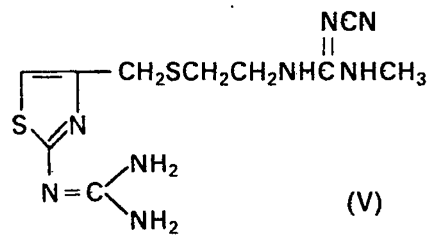

U.S. 4,165,378

(V)

etintidine

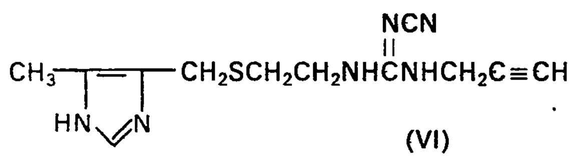

U.S. 4,112,234

(VI)

oxmetidine

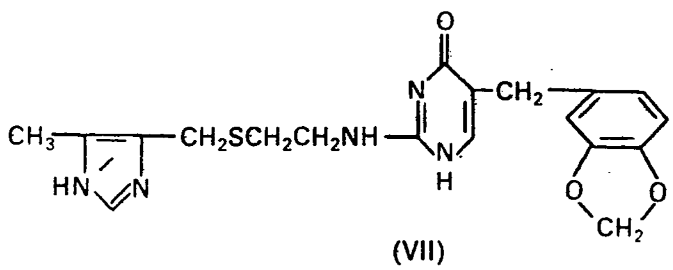

U.S. 4,218,452

(VII)

3- [2-(2-guanidino -4-thiazolylmethylthio)-ethylamino]-4-amino-1,2,5-thiadiazole 1,1-dioxide.

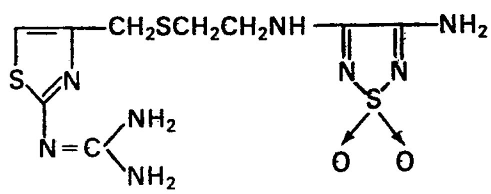

Neth. 8,004,967

(VIII)

2-[2-(5-methyl-4-imidazolylmethylthio)]ethyl-amino-5-(6-methyl-3-pyridylmethyl)-4-pyrimidone

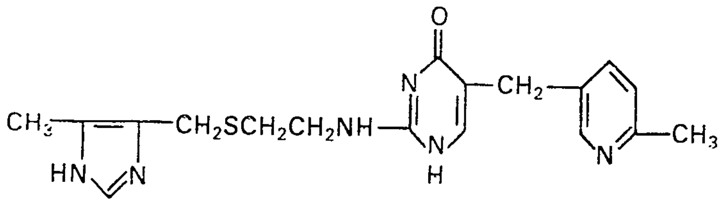

EP 0017679

(IX)

2-[2-(5-dimethylaminomethyl-2-furanylmethylthio)]ethylamino-5-[5-(1,3-benzodioxolyl)methyl]-4-pyrimidone

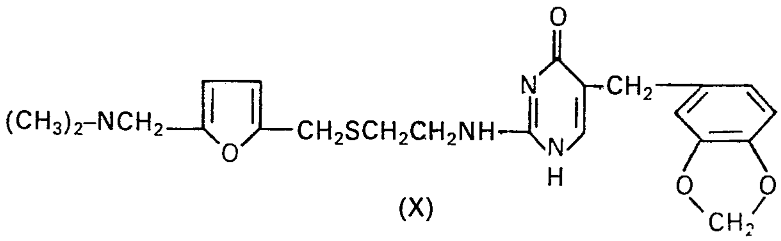

EP 0003677

(X)

2-[2-(5-dimethylaminomethyl-2-furanylmethylthio)]ethylamino-5-(3-pyridylmethyl)-4-pyrimidone

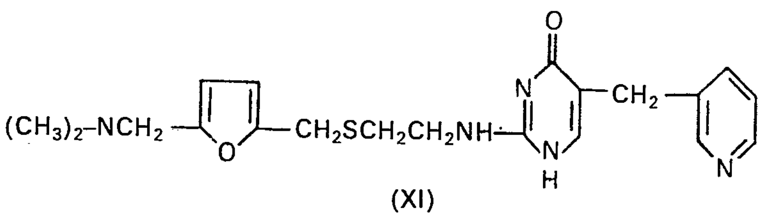

EP 0003677

(XI)

2-[2-(5-dimethylaminomethyl-2-furanylmethylthio)]ethylamino-5-(4-pyridylmethyl)-4-pyrimidone

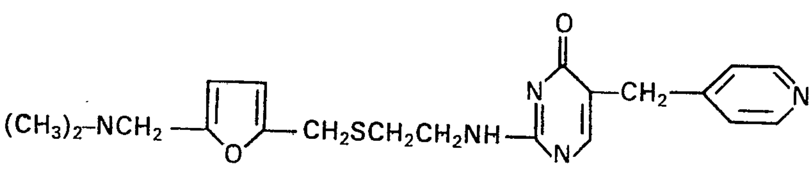

EP 0003677

(XII)

2-[2-(5-(1-pyrrolidino)methyl-2-furanylmethylthio)]ethylamino-5-(6-methyl-3-pyridylmethyl)-4-pyrimidone

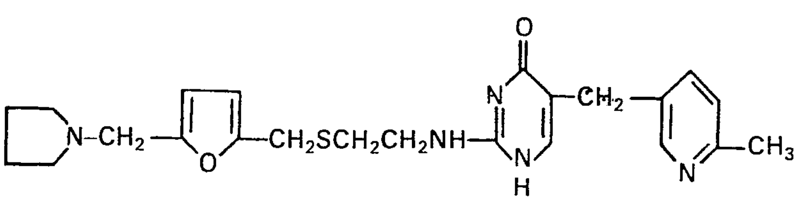

EP 0003677

(XIII)

Any other chemical compound which possesses substantial histamine $H_2$ antagonist activity but no overt toxicity or other detrimental effects to the livestock is also useful as an active ingredient in this invention since it is believed that the increased feed utilization is due to the $H_2$ antagonist activity and not an artifactitious activity.

Among the references in the literature which disclose chemical compounds having histamine $H_2$ antagonist activity from which one skilled in the art can select candidates for being active ingredients in the animal feedstuffs and methods of this invention are: Belgian Patent No. 857 388, 866 156, 866 155, 866 159, 867 105, 867 106, 875 864, 877 889, 867 594, 880 066, 879 024, 882 071; European Patent application No. 1699, 2930, 3640, 6286, 6679, 3677, 10 894; Japanese Patent and Application No. 53-103 468, 53-103 469, 5-4046-780, J5-3132-558, J5-3141-271, J5-5015-414, J5-4106-467; USSR Patent No. 596 577; German Patent Application No. 2 805 827, 2 905 134; British Patent Application No. 80/06354; South African Patent Application No. 80/1151.

The fact that $H_2$ antagonists would enhance feed utilization is in itself surprising since, as is well known in the art, hydrochloric acid causes feed protein to denature as one of the first steps of digestion in many animal species. Of course the main pharmacodynamic action of $H_2$ antagonists is reduction of acid secretion in the digestive tract.

The prime objective of this invention therefore comprises an animal feed composition as described above supplemented by a quantity of one or more chemical compounds as described above which is effective for producing histamine $H_2$ antagonism but which is not toxic to the meat producing animal. The result is an improvement in food utilization and growth rate per unit of feed of the animal.

Exemplary of the effective quantities of active ingredient are those selected from about 5–180 g per ton of feed of metiamide base. A preferred range from which selection may be made is from about 6–40 grams per ton.

The amounts of other chemical compounds having histamine $H_2$ antagonist activity in the claimed feedstuffs are calcultated roughly by comparing the potency of the selected compound with that of metiamide in standard pharmacological tests for such activity for example the urethane anesthetized rat test of gastric secretion using histamine as agonist (i.v. metiamide has an $ID_{50}$ of 1.6 $\mu mol\ kg^{-1}$) or the Heidenhain pouch test in dogs using histamine as agonist (orally metiamide has an $ID_{50}$ of 16 $\mu mol\ kg^{-1}$). Details of screening tests are in the International Symposium on Histamine $H_2$-Receptor Antagonists, London, October 1 and 2, 1973, for example, pages 24 and 297. One should recognize however that the optimum dose level for many particular animal species for any specific compound must be determined by feeding trials to determine such a dose level.

One skilled in the veterinary art will recognize that the cost of the chemical ingredient is an important commercial consideration. While certain $H_2$ antagonist compounds other than metiamide may be more active antagonists, the relatively low chemical cost and good efficacy of metiamide makes the use of this compound as active ingredient beneficial.

The active ingredients are used conveniently in the form of their nontoxic salts with acids acceptable in the veterinary or feed supplement fields whenever the base has unfavorable physical characteristics. The formation of the acid addition salts from the bases is documented in the prior art.

As examples of the livestock feed normally ingested per meat producing animal per day are: sheep 3–4 (1.36–1.81 kg) lbs, feed lot steer 20–25 lbs (9,09–11.36 kg), swine 1–8 lbs (0.45–3.63 kg), poultry 0.03–1 lb (0.01–0.45 kg). Thus this invention comprises feeding from 0.03 to 25 lbs (0,01–11.36 kg) per day per animal of a supplemented livestock feed as described above.

The object of this invention is improving food utilization and growth rate in meat producing animals which are usually immature growing animals such as pigs, cattle, chickens, goats, sheep. The supplemented feeds of this invention which are administering internally to said animals contain a quantity of a chemical compound having histamine $H_2$ receptor antagonistic activity which quantity is nontoxic and sufficient to increase food utilization and growth rate of the animal reflected by increased intake of nutrients. The method of using these compositions is conveniently carried out by administering the active ingredient dispersed uniformly throughout the feed of the livestock for oral administration. The animal is either allowed to feed ad libitum or is fed a supplemented feed ration on a planned schedule.

More specifically the quantity of active $H_2$ antagonist ingredient used in treating meat producing monogastric and ruminant animals will be usually chosen from an effective nontoxic amount selected from the range of about 10 mg–2.5 g per day per animal of metiamide base or its biological equivalent of another compound having $H_2$ antagonist activity with consideration of animal weight and relative activity of the antagonist to metiamide. A very useful range is from about 250 mg–750 mg per day of metiamide base equivalents for pigs and cattle.

Generally speaking, for example, a growing meat producing animal such as a young pig, cow (heifer), chicken, turkey or sheep is fed from 0.03–25 lbs (0.01–11.36 kg) per day of a basal feedstuff containing as active ingredient dispersed therein from about 5–180 grams of compound per ton of feed of a chemical compound having substantial histamine $H_2$ receptor antagonist activity. As noted above, metiamide is the best ingredient known at this thime.

Alternatively, but less desirably, the active ingredient may be mixed with the drinking water of the animal. It may also be administered as a sustained release pellet or bolus implanted intramuscularly or subcutaneously in the growing animal. The hind quarters or ear of a pig or heifer are convenient depositories. This means is used if the active ingredient is not orally effective. In general several daily doses may be combined in such slow release methods of treatment.

In ruminants one skilled in the art might choose to use one of the many ways available to protect the $H_2$ antagonist active ingredient from the complex biological systems of the rumen to transport the chemical for release to the abomasum and lower digestive tract. For example metiamide is imbedded in or coated by a mixture of glyceryl tristearate and a liquid unsaturated higher fatty acid such as oleic acid. The particulate coated material is then mixed with the feedstuff as described above to be fed to the growing ruminant animal.

The following examples are designed to disclose the best mode of this invention as well as to illustrate the practice of the invention but are not intended to limit the scope of application or the number of active ingredients which can be used in the invention.

Example 1

A basal corn based diet is prepared by thoroughly mixing the following ingredients:

| Ingredients | % |
|---|---|
| Ground Corn (to 2 mm size) | 54.62 |
| Soybean Meal (49% protein) | 27.00 |
| Menhaden Fish Meal (60% protein) | 5.00 |
| Meat and Bone Meal | 5.00 |
| Stabilized Animal Fat | 4.00 |
| Dehydrated Alfalfa Meal (17% protein) | 1.25 |
| Dried Whey | 1.00 |
| Limestone | .67 |
| Dicalcium Phosphate Cyphos | .70 |
| Vitamin Premix[a] | .40 |
| Iodized Salt | .23 |
| DL-methionine (98%) | .05 |
| Trace Mineral Mix | .08 |

The corn based ration is mixed with metiamide as follows:

| Test chemical | Dose in feed (ppm) | Corn basal (grams) | Compound (grams) |
|---|---|---|---|
| A Control | 0 | 100,000 | 0 |
| B (Metiamide) | 4 | 99,99.6 | 0.4 |
| C (Metiamide) | 8 | 99,999.2 | 0.8 |
| D (Metiamide) | 16 | 99,998.4 | 1.6 |

The supplemented ration was fed to 3 day old cockerels with 16 pens each containing 8 chicks used at each dose level. The chicks were allowed to feed at will. The following results were obtained:

| Treatment | ppm | Chick Weight-17 days | % Improvement |
|---|---|---|---|
| Control | 0 | 346.1 | – |
| Metiamide | 4 | 342.0 | −1.2 |
| Metiamide | 8 | 349.2 | +0.9 |
| Metiamide | 16 | 354.4 | +2.4 |

The above data was a confirmation of positive results obtained over a dose range earlier in which 4 pens of 8 chicks were used at each dose level with the following results:

| Treatment | ppm | Chick Weight-17 days | % Improvement |
|---|---|---|---|
| Control | 0 | 301.8 | – |
| Metiamide | 8 | 316.9 | +5.0 |
| Metiamide | 40 | 311.5 | +3.2 |
| Metiamide | 200 | 307.3 | +1.8 |

Exemple 2

An example of a suitable premix is as follows:

| | |
|---|---|
| Metiamide | 200 g |
| Ground yellow corn | 20 lbs (9.09 kg) |

Example 3

In the field the active ingredients may be administered by means of salt or molasses blocks. A typical block may be prepared using the following conditions:

| Ingredients | Weight percent |
|---|---|
| Dried cane molasses | 39.50 |
| Ground soybean hulls | 29.90 |
| Metiamide | 5.00 |
| Granulated salt | 21.59 |
| Trace minerals and vitamins | 0.20 |
| Stabilized animal fat | 1.11 |
| Moisture | 2.66 |

Example 4

| Ingredients | Weight percent |
|---|---|
| Metiamide | 300 mg |
| Calcium sulfate, dihydrate | 70 mg |
| Gelatin | 4 mg |
| Magnesium stearate | 1 mg |
| Talc | 2 mg |

The $H_2$ antagonist and calcium sulfate, dihydrate are mixed and passed through a No. 40

standard mesh screen. The screened mixture is then granulated with hot 15 percent gelatin solution, screened through a No. 10 mesh screen and dried overnight at 120 °F (48.84 °C). The granules are again screened through a No. 40 mesh screen and mixed with the magnesium stearate and talc. The granules are compressed into implants using a 1/8 inch ($3.175 \cdot 10^{-3}$ m) flat face punch and die. One implant is administered intramuscularly to a feed lot yearling.

Example 5

A basal swine feed mixture of standard 16% protein, corn, soybean meal ration fortified with vitamins and minerals and having dispersed therein metiamide at the concentration of 100 g per ton is fed to 20–25 lb (9.09–11.36 kg). Hampshire swine.

Example 6

| | % by weight |
|---|---|
| Alfalfa hay, leafy ground | 65 |
| Corn shelled | 12 |
| Barley | 9 |
| Soybean meal | 10 |
| Molasses | 2.5 |
| Bone meal | 1 |
| Salt | 0.5 |
| Metiamide | 25 g/ton of feed |

The above mixture is prepared and is fed to feed lot lambs ad libitum.

Example 7

Using the same methods and base corn feed as in Example 1 but with 8 pens and 8 chicks at each test level the following data were obtained:

| Chemical | Dose in Feed (ppm) | Weight of Animal Day 10 | Day 17 | Feed (grams)/Gain (grams) Day 3–10 | Day 10–17 |
|---|---|---|---|---|---|
| Control Corn | – | 183.0 g | 359.7 g | 1.97 g/g | 1.84 g/g |
| | | Percent of Control | | | |
| Ranitidine | 8 | 99.9 | 108.1 | 93.0 | 92.1 |
| Tiotidine | 8 | 99.9 | 97.3 | 93.9 | 107.0 |

Example 8

The diets outlined hereafter containing 6, 18 and 54 parts per million of metiamide were fed ad lib. to 96 crossbred Yorkshire pigs weighing 15–25 kg at the beginning of the test distributed in 24 pens with control groups:

| | Diet Starter | Grower | Finisher |
|---|---|---|---|
| Ingredients (%) | | | |
| Ground corn | 66.0 | 70.60 | 79.25 |
| Soybean oil meal (44%) protein) | 26.00 | 22.00 | 13.35 |
| Dehydrated alfalfa meal | 5.00 | 4.50 | 4.50 |
| Vitamin and mineral supplement | 2.75 | 2.75 | 2.75 |
| Calcium propionate | 0.15 | 0.15 | 0.15 |
| Calculated Analysis (%) | | | |
| Crude protein | 18.11 | 16.7 | 13.62 |
| Lysine | 0.96 | 0.84 | 0.61 |

The pigs were feed non-supplemented starter ration for 14 days, supplemented grower ration to noncontrol pens for 3 weeks then finisher ration until staggered sacrifice times.

At 6, 18 and 54 parts per million of metiamide growth was stimulated 5–7% over control lots. 18 and 54 parts per million improved feed utilization efficiency during the two growing-finishing phases 4.3% over control. Younger animals grew at a faster rate than more mature animals.

The test demonstrates the positive effect on growth and feed utilization using compounds having $H_2$ receptor antagonist activity as active ingredients especially in feeder pig and veal calf production.

The other compounds having histamine $H_2$ antagonist activity mentioned above by structure and generic name may be substituted in the foregoing examples for metiamide in quantities from 1/2 to 1/20 those of metiamide. Other com-

pounds having histamine $H_2$ antagonist activity may be similarly used by comparing their oral and parenteral activity with that of metiamide in standard pharmacological procedures used in the art for demonstrating $H_2$ antagonist activity then adjusting the dosage levels in the particular animal species in question using the methods and compositions of this invention described herein.

Also the active ingredients of the compositions of this ingredient may be combined with other livestock feed additions known to the art such as an antibiotic for example virginimycin, monensin or aureomycin, an anthelmintic or a methanogenesis inhibitor such as amicloral.

**Claims for the Contracting States: BE, CH, DE, FR, GB, IT, LI, LU, NL, SE**

1. A feed composition for meat producing animals comprising a quantity of a chemical compound having histamine $H_2$ receptor antagonist activity which is sufficient to improve the food utilization of said animals dispersed in a premix or complete livestock feed ration.

2. The feed of claim 1 in which the chemical compound is metiamide, cimetidine, ranitidine, etintidine or oxmetidine.

3. The feed of claim 1 in which the chemical compound is metiamide.

4. The feed of claim 1 in which the feed is one adapted for poultry and the chemical compound is metiamide.

5. The feed of claim 1 in which the feed is one adapted for swine and the chemical compound is metiamide.

6. The feed of claim 1 in which the chemical compound is ranitidine.

7. The feed of claims 1–6 in which the amount of said chemical compound is selected from the range of 5–180 grams per ton of feed.

8. The feed of claim 1 in which the feed ix a premix feed comprising a feed ration combined with 0.5–3% by weight of the said chemical compound.

9. The feed of claim 1 in which said chemical compound is metiamine or ranitidine which is present at a concentration to give from 6–40 grams per ton of complete feed.

10. A method of improving food utilization in meat producing animals which comprises administering to said animals an effective but non-toxic quantity of a chemical compound having histamine $H_2$-receptor antagonist activity.

**Claims for the contracting State: AT**

1. A process for preparing a feed composition for meat producing animals comprising a quantity of a chemical compound having histamine $H_2$ receptor antagonist activity which is sufficient to improve the food utilization of said animals dispersed in a premix or complete livestock feed ration which comprises dispersing that quantity of the chemical compound in the premix or feed ration.

2. The process of claim 1 in which the chemical compound is metiamide, cimetidine, ranitidine, etintidine or oxmetidine.

3. The process of claim 1 in which the chemical compound is metiamide.

4. The process of claim 1 in which the feed is one adapted for poultry and the chemical compound is metiamide.

5. The process of claim 1 in which the feed is one adapted for swine and the chemical compound is metiamide.

6. The process of claim 1 in which the chemical compound is ranitidine.

7. The process of claims 1–6 in which the amount of said chemical compound is selected from the range of 5–180 grams per ton of feed.

8. The process of claim 1 in which the feed is a premix feed comprising a feed ration combined with 0.5–3% by weight of the said chemical compound.

9. The process of claim 1 in which said chemical compound is metiamide or ranitidine which is present at a concentration to give from 6–40 grams per ton of complete feed.

**Patentansprüche für die Vertragsstaaten: BE, CH, DE, FR, GB, IT, LI, LU, NL, SE**

1. Futterzusammensetzung für fleischproduzierende Tiere, umfassend eine zur Verbesserung der Futterverwertung der Tiere ausreichende Menge einer chemischen Verbindung mit Histamin $H_2$-Rezeptor-Antagonistenwirkung, verteilt in einem Vorgemisch oder in der vollständigen Viehfutterration.

2. Futter nach Anspruch 1, in dem die chemische Verbindung Metiamid, Cimetidin, Ranitidin, Etintidin oder Oxmetidin ist.

3. Futter nach Anspruch 1, in dem die chemische Verbindung Metiamid ist.

4. Futter nach Anspruch 1, in dem das Futter ein für Geflügel geeignetes ist und die chemische Verbindung Metiamid ist.

5. Futter nach Anspruch 1, in dem das Futter ein für Schweine geeignetes ist und die chemische Verbindung Metiamid ist.

6. Futter nach Anspruch 1, in dem die chemische Verbindung Ranitidin ist.

7. Futter nach Anspruch 1 bis 6, in dem die Menge der chemischen Verbindung im Bereich von 5 bis 180 g/Tonne Futter liegt.

8. Futter nach Anspruch 1, in dem das Futter ein Futter-Vorgemisch ist, das eine Futterration in Verbindung mit 0,5 bis 3 Gewichtsprozent der chemischen Verbindung umfasst.

9. Futter nach Anspruch 1, in dem die chemische Verbindung Metiamin oder Ranitidin ist, welches in einer Konzentration vorhanden ist, die 6 bis 40 g/Tonne des vollständigen Futters ergibt.

10. Verfahren zur Verbesserung der Futterverwertung bei fleischproduzierenden Tieren, umfassend die Verabreichung einer wirksamen aber nicht toxischen Menge einer chemischen Verbindung mit Histamin $H_2$-Rezeptor-Antagonistenwirkung an die Tiere.

**Patentansprüche für den Vertragsstaat: AT**

1. Verfahren zur Herstellung einer Futterzusammensetzung für fleischproduzierende Tiere, welche eine zur Verbesserung der Futterverwertung dieser Tiere ausreichende Menge einer chemischen Verbindung mit Histamin $H_2$-Rezeptor-Antagonistenwirkung, verteilt in einem Vorgemisch oder der vollständigen Viehfutterration umfasst, dadurch gekennzeichnet, dass die Menge der chemischen Verbindung in dem Vorgemisch oder in der Futterration verteilt wird.

2. Verfahren nach Anspruch 1, in dem die chemische Verbindung Metiamid Cimetidin, Ranitidin, Etintidin oder Oxmetidin ist.

3. Verfahren nach Anspruch 1, in dem die chemische Verbindung Metiamid ist.

4. Verfahren nach Anspruch 1, in dem das Futter ein für Geflügel geeignetes ist und die chemische Verbindung Metiamid ist.

5. Verfahren nach Anspruch 1, in dem das Futter ein für Schweine geeignetes ist und die chemische Verbindung Metiamid ist.

6. Verfahren nach Anspruch 1, in dem die chemische Verbindung Ranitidin ist.

7. Verfahren nach Anspruch 1 bis 6, in dem die Menge der chemischen Verbindung im Bereich von 5 bis 180 g/Tonne Futter liegt.

8. Verfahren nach Anspruch 1, in dem das Futter ein Futter-Vorgemisch ist, das eine Futterration mit 0,5 bis 3 Gewichtsprozent der genannten chemischen Verbindung umfasst.

9. Verfahren nach Anspruch 1, in dem die chemische Verbindung Metiamid oder Ranitidin ist, welche in einer Konzentration vorhanden sind, die 6 bis 40 g/Tonne vollständiges Futter ergibt.

**Revendications pour les Etats contractants:**
**BE, CH, DE, FR, GB, IT, LI, LU, NL, SE**

1. Composition alimentaire pour animaux producteurs de viande qui comprend, dispersé dans un prémélange ou une ration alimentaire complète, un composé chimique ayant une activité antagoniste des récepteurs $H_2$ à l'histamine en quantité suffisante pour améliorer le rendement nutritif desdits animaux.

2. Aliment suivant la revendication 1, dans lequel le composé chimique est le métiamide, la cimétidine, la ranitidine, l'étintidine ou l'oxmétidine.

3. Aliment suivant la revendication 1, dans lequel le composé chimique est le métiamide.

4. Aliment suivant la revendication 1, dans lequel l'aliment est un aliment convenant pour la volaille et dans lequel le composé chimique est le métiamide.

5. Aliment suivant la revendication 1, dans lequel l'aliment est un aliment convenant pour le porc et dans lequel le composé chimique est le métiamide.

6. Aliment suivant les revendications 1, dans lequel le composé chimique est la ranitidine.

7. Aliment suivant les revendications 1 à 6, dans lequel la quantité dudit composé chimique est choisie dans l'intervalle compris entre 5 et 180 grammes par tonne d'aliment complet.

8. Aliment suivant la revendication 1, dans lequel l'aliment est un prémélange alimentaire comprenant une ration alimentaire associée avec 0,5 à 3% en poids dudit composé chimique.

9. Aliment suivant la revendication 1, dans lequel ledit composé est le métiamide ou la ranitidine qui est présent à une concentration de 6 à 40 grammes par tonne d'aliment complet.

10. Méthode pour améliorer le rendement nutritif des animaux producteurs de viande qui comprend l'administration auxdits animaux d'une quantité efficace mais non toxique d'un composé chimique ayant une activité antagoniste des récepteurs $H_2$ à l'histamine.

**Revendications pour l'Etat contractant: AT**

1. Procédé de préparation d'une composition alimentaire pour animaux producteurs de viande comprenant, dispersé dans un prémélange ou une ration alimentaire complète, un composé chimique ayant une activité antagoniste des récepteurs $H_2$ à l'histamine en quantité suffisante pour améliorer le rendement nutritif desdits animaux, qui comprend la dispersion de cette quantité de composé chimique dans le prémélange ou la ration alimentaire.

2. Procédé suivant la revendication 1, dans lequel le composé chimique est le métiamide, la cimétidine, la ranitidine, l'étintidine ou l'oxmétidine.

3. Procédé suivant la revendication 1, dans lequel le composé chimique est le métiamide.

4. Procédé suivant la revendication 1, dans lequel l'aliment est un aliment convenant pour la volaille et dans lequel le composé chimique est le métiamide.

5. Procédé suivant la revendication 1, dans lequel l'aliment est un aliment convenant pour le porc et dans lequel le composé chimique est le métiamide.

6. Procédé suivant la revendication 1, dans lequel le composé chimique est la ranitidine.

7. Procédé suivant les revendications de 1 à 6, dans lequel la quantité dudit composé chimique est choisie dans l'intervalle compris entre 5 et 180 grammes par tonne d'aliment complet.

8. Procédé suivant la revendication 1, dans lequel l'aliment est un prémélange alimentaire comprenant une ration alimentaire associée avec 0,5 à 3% en poids dudit composé chimique.

9. Procédé suivant la revendication 1, dans lequel ledit composé est le métiamide ou la ranitidine qui est présent à une concentration de 6 à 40 grammes par tonne d'aliment complet.